Europäisches Patentamt

European Patent Office

Office européen des brevets

(11) Veröffentlichungsnummer: **0 293 504 B1**

# EUROPÄISCHE PATENTSCHRIFT

(45) Veröffentlichungstag der Patentschrift: **20.02.91**

(51) Int. Cl.⁵: **A61B 5/00, A61B 5/02**

(21) Anmeldenummer: **87108024.8**

(22) Anmeldetag: **03.06.87**

(54) Verfahren zur Bestimmung der Perfusion.

(43) Veröffentlichungstag der Anmeldung:
**07.12.88 Patentblatt 88/49**

(45) Bekanntmachung des Hinweises auf die
Patenterteilung:
**20.02.91 Patentblatt 91/08**

(84) Benannte Vertragsstaaten:
**DE FR GB IT NL**

(56) Entgegenhaltungen:
**WO-A-86/05674**
**US-A- 4 167 331**

**IEEE TRANSACTIONS ON BIOMEDICAL ENGI-
NEERING BME-33, Nr. 8, August 1986, Seiten
795-798, New York, US; H. SHIMAZU et al.:
"Noninvasive Measurement of the Volume
Elastic Modulus in Finger Arteries Using
Photoelectric Plethysmography"**

**Medical Instrumentation: Application and
Design, Webster, Houghton Mifflin Com. Boston, 1978, Seite 94**

(73) Patentinhaber: **Hewlett-Packard GmbH
Herrenberger Strasse 130 Postfach 14 30
D-7030 Böblingen(DE)**

(72) Erfinder: **Noller,Friedemann,Dr.Ing.Dipl.Phys.
Neusatzstr.22
D-7033 Herrenberg(DE)**
Erfinder: **Forstner,Klaus,Dipl.Ing.
Aspergerstr. 6
D-7146 Tamm(DE)**

**Beschreibung**

Die Erfindung betrifft ein Verfahren zur Bestimmung der Perfusion gemäß der Gattung des Hauptanspruchs.

Die Pulsation des arteriellen Blutes bewirkt eine pulsierende Volumenänderung der Arterien. Wird nun das von einer Lichtquelle ausgesandte Licht durch ein durchblutetes Körperteil gesandt, so erfährt das Licht eine von der Pulsation abhängige Dämpfung. Die daraus resultierende Intensitätsänderung des mittels eines Sensors empfangenen Lichts kann zum Beispiel zur Bestimmung der Sauerstoffsättigung des Blutes herangezogen werden. Den Zusammenhang zwischen der Lichtabsorption im Gewebe und der Beschaffenheit und der Dicke des vom Licht durchstrahlten Gewebes gibt das Lambert-Beersche Gesetzan bzw. dessen Modifikation zur Erfassung von Streueffekten.

Aus der US-A-4,109,643 ist eine Einrichtung zur Messung der Perfusion bekannt, die eine lichtemittierende Diode für eine Messung im Bereich der Fingerkuppe verwendet. Die lichtemittierende Diode ist zu diesem Zweck an der einen Seite einer Fingerkuppe angebracht, während ein photoempfindlicher Sensor an der gegenüberliegenden Seite angeordnet ist. Mittels einer elektronischen Auswerteeinrichtung kann bei dieser bekannten Anordnung eine tendenzielle Änderung der Perfusion festgestellt werden, jedoch kann keine Angabe gemacht werden, welche Volumenänderung das arterielle Blut bei einer Pulsation prozentual erfährt. Da vor Operationen beispielsweise aufgrund eines Schockzustandes am Patienten eine normale Perfusion nicht mehr vorliegt, kann im Wege der Messung der Änderung der Perfusion keine exakte Aussage über den Zustand der Patienten während der Operation gemacht werden. Die Messung der Perfusionsänderung ist nur dann sinnvoll, wenn für den Patienten ein korrekter Bezugswert vor dem Eintreten einer Perfusionsbeeinträchtigung ermittelt werden konnte. Außerdem kann die Perfusionsmessung physiologisch als Indikator für den Stresszustand eines Patienten herangezogen werden.

Aus der US-A-4,167,331 ist ferner die Messung eines "Perfusionsindex" bekannt. Auch bei der dort beschriebenen Meßmethode handelt es sich allerdings um eine unskalierte, d.h. unquantifizierbare Messung mit den oben beschriebenen Nachteilen. Aus den "IEEE Transactions on Biomedical Engineering" BME-33, Nr. 8, August 1986, Seite 795 ff. ist schließlich auch eine plethysmographische Meßmethode bekannt, d.h. eine Meßmethode, in deren Meßergebnis auch die Perfusion eingeht. Der dort ermittelte Meßwert hängt jedoch auch stark von der Sauerstoffsättigung des Blutes ab, so daß sich kein zuverlässiger und quantifizierbarer Wert für die Perfusion angeben läßt.

Der Erfindung liegt die Aufgabe zugrunde, ein Verfahren zur Bestimmung der Perfusion anzugeben, mit dem die Perfusion quantifiziert werden kann.

Die Lösung dieser Aufgabe wird bei einem Verfahren der eingangs genannten Gattung durch die im kennzeichnenden Teil des Hauptanspruchs angegebenen Merkmal erhalten. Die Volumenänderung des arteriellen Blutes wird durch sehr kleine Dickenänderungen hervorgerufen, so daß Gleichungsteile, bei denen die Dickenänderung mit höherer Ordnung auftritt, vernachlässigt werden können. Somit ergibt sich näherungsweise, daß die Volumenänderung, bezogen auf das Meßvolumen, ungefähr der dreifachen Dickenänderung des mittleren Gesamtquerschnitts der arteriellen Gefäße, bezogen auf die Dicke des durchbluteten Meßvolumens, ist.

Das die Lichtabsorption nicht nur vom Volumen des durchbluteten Gewebes abhängig ist, sondern auch von der Konzentration der lichtabsorbierenden Bestandteile des Blutes, muß z.B. die Hämoglobin-bzw. Oxyhämoglobinkonzentration empirisch vorher ermittelt werden, damit mittels einer Messung bei einer einzigen Wellenlänge aus der Lichtabsorption die Perfusion bestimmt werden kann.

Durch Mehrfachmessung können auch weitere Parameter der zugrundeliegenden Gleichung bestimmt bzw. eliminiert werden. Beispielsweise kann durch Messung bei drei unterschiedlichen Wellenlängen die Sauerstoffsättigung, die Perfusion und eine weitere Größe bestimmt werden. Die Hämoglobinkonzentration kann näherungsweise für Frauen mit 140 g/l $\simeq$ 8,7 mMol/l und für Männer mit 160 g/l $\simeq$ 9,94 mMol/l angenommen werden. Bei Neugeborenen beträgt die Hämoglobinkonzentration durchschnittlich 200 g/l $\simeq$ 12,42 mMol/l.

Die Erfindung wird nachfolgend anhand der Zeichnungen näher erläutert. Es zeigen:

Figur 1     eine auf eine Fingerkuppe aufgesteckte Meßkappe mit zwei lichtemittierenden Dioden und einem Photoempfänger,

Figur 2     den grundsätzlichen Aufbau eines mit Arterien und Venen durchzogenen Gewebes,

Figur 3     einen Gewebeabschnitt, an dem Licht eingestrahlt wird, welches teilweise das Gewebe durchdringt und teilweise reflektiert wird,

Figur 4     den am Photoempfänger auftretenden, durch die Pulsation des arteriellen Blutes beeinflußten Stromverlauf,

Figur 5    den Extinktionskoeffizienten für unterschiedliche Sauerstoffsättigung in Abhängigkeit von der Wellenlänge des eingestrahlten Lichtes,

Figur 6    ein Blockschaltbild einer Perfusions-Meßeinrichtung und

Figur 7    die analoge Empfangselektronik für die in Figur 6 verwendete optische Meßeinrichtung.

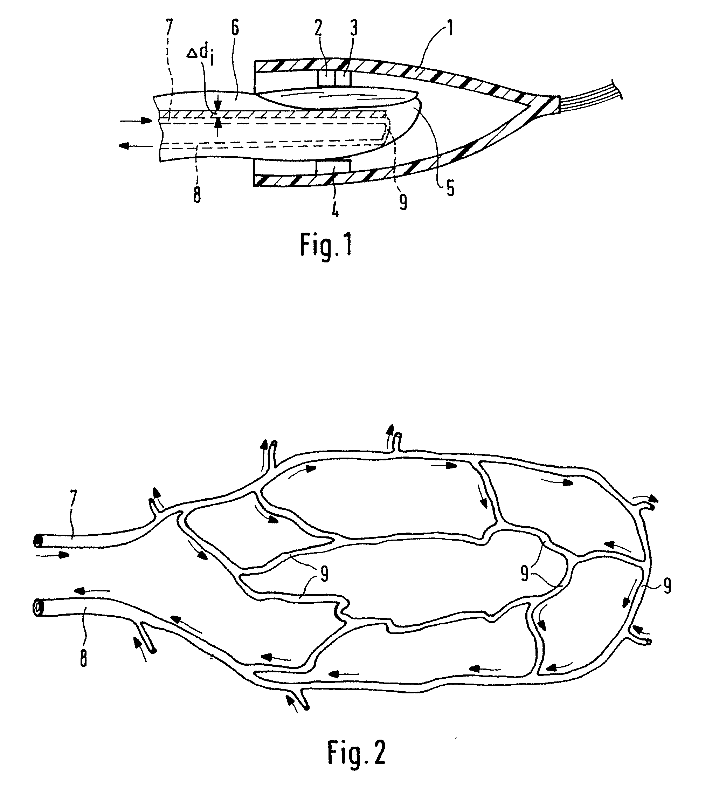

In Figur 1 ist eine Meßkappe 1 mit zwei lichtemittierenden Dioden 2, 3 und einem Photoempfänger 4 auf eine Fingerkuppe 5 aufgesteckt. Die im Gewebe 6 verlaufenden Arterien 7 und Venen 8 sind hier mit unterbrochenen Linien schematisch angedeutet. Zwischen Arterien 7 und Venen 8 befinden sich unter anderem Kapillaren 9, die druckmindernd wirken, so daß praktisch nur in den Arterien 7 eine Pulsation und eine damit verbundene, pulsabhängige Volumenänderung auftritt.

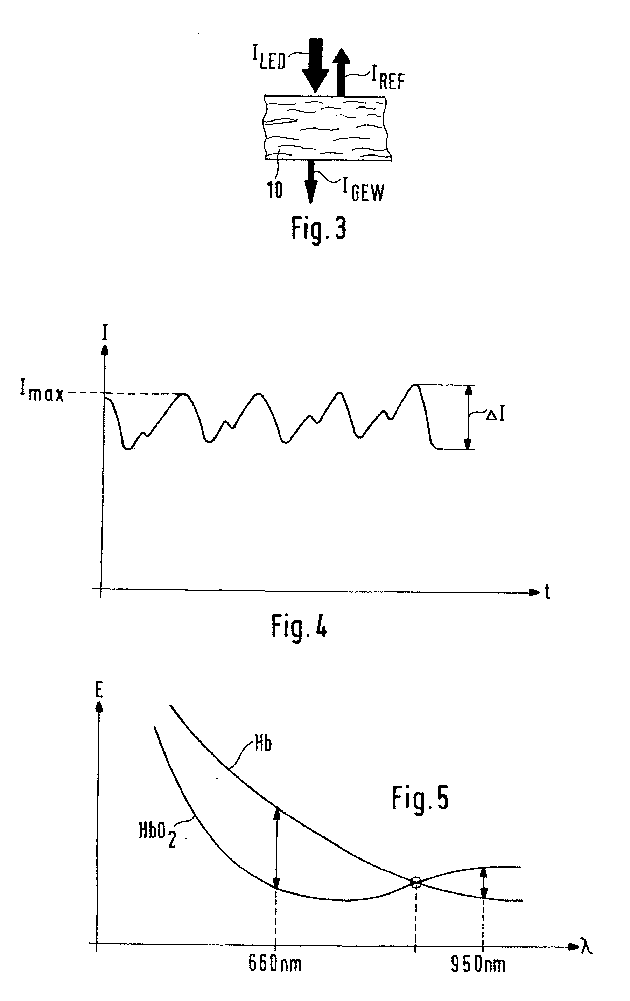

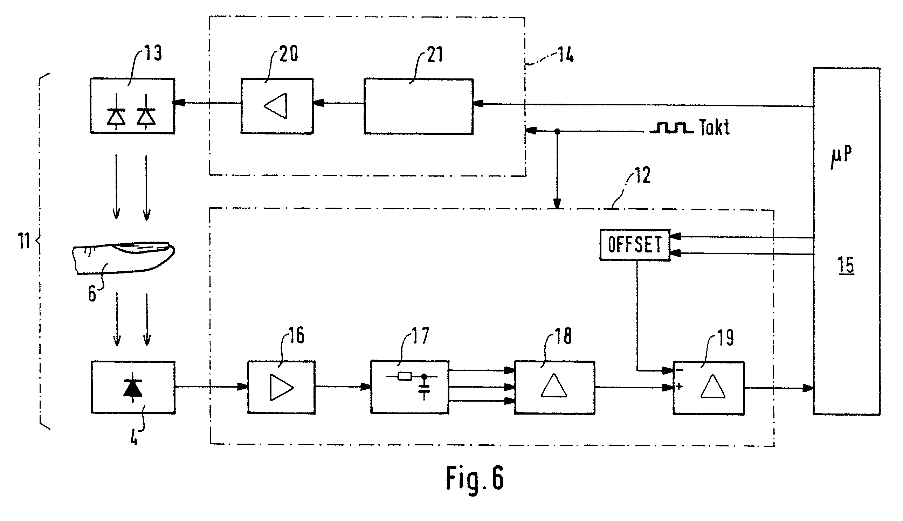

Die lichtemittierenden Dioden 2, 3 senden Licht mit unterschiedlicher Wellenlänge, beispielsweise mit 660 nm und mit 950 nm zum Photoempfänger 4 aus. Der Photoempfänger 4 wandelt das empfangene Licht in ein Stromsignal um, wie es in Figur 4 dargestellt ist. Das Stromsignal wird in einer wie in Figur 6 dargestellten Auswerteschaltung zur Bestimmung der Perfusion ausgewertet.

In Figur 2 ist die Struktur eines durchbluteten Gewebeabschnitts dargestellt. Durch die Arterie 7 strömt das Blut ein und gelangt über Kapillaren 9 und Zellen zur Vene 8, über die es wieder abfließt.

Wird nun ein durchbluteter Gewebebereich von einer Lichtquell bestrahlt, so erfährt das Licht im Gewebe eine Absorbtion, die in erster Näherung vom molekularen Extinktionskoeffizienten E, der Konzentration c des gelösten Farbstoffes und der Dicke d der durchstrahlten Schicht abhängt.

In Figur 3 ist ein Gewebeabschnitt 10 dargestellt, der von einem dem Strom $I_{LED}$ entsprechenden Licht bestrahlt wird, wobei das Gewebe eine Reflexion bzw. eine Transmission bewirkt bzw. zuläßt, die die Intensität

$$I_{GEW} = I_{LED} \times e^{-Ecd} \qquad (1)$$

wobei

$I_{GEW} =$    Intensität nach dem Absorber am Detektor

$I_{LED} =$    Intensität vor dem bzw. ohne Absorber

$E =$    molarer Extinktionskoeffizient

    $E(\lambda)$ [l/ Mol x cm]

$c =$    molare Konzentration der absorbierenden Substanz [Mol/l]

$d =$    Dicke des Absorbers [cm]

Gleichung (1) ist das Lambert-Beersche Gesetz, wobei für strenge Gültigkeit paralleles, monochromatisches Licht mit einem optisch homogenen Absorber vorausgesetzt wird. Die reale Zusammensetzung eines durchbluteten Körperteils (z.B. Fingerkuppe) erfordert beispielsweise für die Lichtabsorbtion des blutes die Berücksichtigung der diskreten Blutkörperchen als Streuzentren. Daher ist für genauere Betrachtungen z.B. die Lichtquantendiffusion zu berücksichtigen, welche anstelle der Extinktion als konstante Größe bei gegebener Wellenlänge eine effektive Extinktion $E_{eff}$ ($\lambda$, $C_{Hb}$, $C_{HbO2}$ ...) vorsieht, welche weitere Abhängigkeiten, z.B. die Hämoglobinkonzentration berücksichtigt.

Für die weiteren Ableitungen gelte zwecks vereinfachter Darstellung weiterhin die Gleichung (1).

Für die gesamte Gewebeabsorption gilt für den Fall eines pulsierend durchbluteten Körperteils folgende Separation in einen zeitabhängigen und zeitunabhängigen Anteil, entsprechend dem Prinzip der Pulsoximetrie.

$$Ecd = E_{Hb} \times c_{Hb} \times \Delta d(t) + E_{HbO2} \times C_{HbO2} \times \Delta d(t) + E_N \times c_N \times d_N \qquad (2)$$

wobei

$E_{HbO2} \times c_{HbO2} \times \Delta d(t) =$    zeitabhängiger Anteil des Oxyhämoglobins

$\Delta d(t) =$    mittlere Gesamtquerschnittsverbreiterung der arteriellen Gefäße,

$E_N \times c_N \times d_N =$    zeitunabhängige Absorbtion des restlichen Körperteils

Damit folgt für den zeitunabhängigen Anteil der Intensität am als Photoempfänger ausgebildeten Detektor

$$Imax = I_{LED} \times e^{-E_N \times c_N \times d_N} \qquad (3)$$

und für den zeitabhängigen, pulsierenden Anteil

$$\Delta I(t) = Imax - I_{GEW} = Imax(1 - e^{-(E_{Hb} \times c_{Hb} + E_{HbO2} \times c_{HbO2}) \times \Delta d(t)}) \qquad (4)$$

Verallgemeinert folgt für eine aus n optisch aktiven Komponenten bestehende Blutzusammensetzung aus Gleichung (4)

$$\ln(1 - \Delta I/Imax \mid_{\lambda i}) = -(E_{1i} \times c_1 + E_{2i} \times c_2 + E_{ni} \times c_n) \Delta d \qquad (5) \text{ wobei}$$

$E_{ni} =$    molekularer Extinktionskoeffizient des Absorbers n bei Wellenlänge $\lambda_i$,

$c_n =$    molekulare Konzentration des Absorbers n im pulsierenden Volumen,

$\Delta d$ = mittlere Gesamtquerschnittsverbreiterung der arteriellen Gefäße.

Für kleine Intensitätsänderungen gilt näherungsweise

$\Delta I/I_{max} \simeq \ln(1 - \Delta I/I_{max})$ für $\Delta I \ll I_{max}$ (6)

d.h. $\Delta I/I_{max}$ ist proportional zur mittleren Gesamtquerschnittsverbreiterung $\Delta d$ bzw. der Perfusionsdicke $\Delta d$.

Entsprechend der Anzahl der unbekannten Größen in Gleichung (5) ist mit der entsprechenden Anzahl von Meßwerten mit den Wellenlängen $\lambda_i$ das Gleichungssystem bestimmbar.

Im Falle eines 2-Wellenlängensystems kann beispielsweise die Sauerstoffsättigung als

$S = C_{HbO2}/[Hb]$ (7)

bestimmt werden, wobei

$[HB] = c_{Hb} + c_{HbO2}$

der Gesamthämoglobinkonzentration entspricht.

Als weitere Größe kann die Perfusionsdicke $\Delta d$ bestimmt werden, falls die Hämoglobinkonzentration als bekannt vorausgesetzt werden kann.

$$\Delta d = \frac{(E_{HbO2} - E_{Hb})_1 \times \ln(1 - \dfrac{\Delta I}{I_{max}})_2 - (E_{HbO2} - E_{Hb})_2 \times \ln(1 - \dfrac{\Delta I}{I_{max}})_1}{[Hb] \, [E_{HbO2}(2) \times E_{Hb}(1) - E_{HbO2}(1) \times E_{Hb}(2)]} \quad (8)$$

wobei z.B.

(1) = Wellenlänge 660 nm

(2) = Wellenlänge 950 nm

$E_{Hb}(1)$ = 820 l/Molcm

$E_{HbO2}(1)$ = 100 l/Molcm

$E_{Hb}(2)$ = 100 l/Molcm

$E_{HbO2}(2)$ = 260 l/Molcm

$[Hb]$ = 9 mMol/l

Falls ein isobestischer Punkt $E_{Hb} = E_{HbO2}$ gewählt wird, reicht zur Perfusionsbestimmung auch ein Meßwert $\Delta I/I_{max}$. Mit den Meßwerten

$\Delta I/I_{max}(1) = 0,06$

$\Delta I/I_{max}(2) = 0,1$

folgt für die Perfusionsdicke

$\Delta d = 0,046$ cm

Eine andere mögliche Definition für die Perfusion ist die relative Volumenänderung $\Delta V/V$, wobei $\Delta V$ der Blutvolumenänderung pro Pulsation entspricht und $V$ den zugehörigen Meßvolumen zwischen Lichtquelle und Detektor entspricht. Falls die Perfusionsdickenänderung $\Delta d \ll d$ ist, wobei $d$ der gesamten Absorberdicke (z.B. Finger) entspricht, gilt bei allseitig gleichmäßiger Verbreiterung

$(V_{max} - V)/V = \Delta V/V \simeq 3 \, \Delta d/d$ für $\Delta d \ll d$. (9)

Die Fingerdicke beträgt durchschnittlich 1 cm, so daß Gleichung (9) gut erfüllt ist.

Mit Gleichung (3) kann $d$ bestimmt werden, falls $E_N \times c_N$ und $I_{LED}$ bekannt sind.

Mit $I_{LED}(1) = \alpha_1 \times I_0$ und $I_{LED}(2) = \alpha_2 \times I_0$ gilt

$$d_N = \ln\left(\frac{\alpha_1 \times Imax(2)}{\alpha_2 \times Imax(1)}\right) \frac{1}{E_N \times c_N(1) - E_N \times c_N(2)} \quad (10)$$

Die einmal ermittelte Differenz $E_N \times c_N(1) - E_N \times c_N(2)$ bei konstanter Absorberdicke $d_N$ bleibt bei weitgehend homogener Organzusammensetzung (z.B. Ohrläppchen) weitgehend konstant, ebenso die aus den Strom-Lichtintensitätskennlinien einmal ermittelten Werte $\alpha_1$ und $\alpha_2$, die z.B. ohne Absorber ermittelt wurden.

Für den Fall einer punktförmigen Lichtquelle, insbesondere bei der Messung an der Fingerkuppe, folgt mit den Lichtintensitäten $I_{LED}$ bzw. $Imax$ im Abstand $d_N$ in erster Näherung

$$d_N = \sqrt{\dfrac{k \times I_{LED}}{Imax}} \qquad (11)$$

wobei k mit der einmal ermittelten Strom-Lichtintensitätskennlinie bestimmt wird und näherungsweise als Konstante zu betrachten ist.

Damit läßt sich nach Gleichung (10) bzw. (11) auch fortlaufend die Dicke d des durchbluteten Körperteils messen. Dies kann auch als Indikator für eine richtige Sensorposition dienen. Schließlich kann damit die Perfusion nach Gleichung (9) als normierte Größe der Blutvolumenänderung pro Pulsation am entsprechenden Körperteil ermittelt werden.

Im obigen Beispiel folgt mit $d_N = 1$ cm

$$\Delta V/V = 13,8 \ \%$$

Das in Figur 6 dargestellte Blockschaltbild zeigt die optische Meßeinrichtung 11, deren Photoempfänger 4 mit einer Auswerteschaltung 12 verbunden ist. Als Lichtquelle 13 können beispielsweise drei lichtemittierende Dioden unterschiedlicher Wellenlänge vorgesehen sein, die von einer LED-Ansteuerung 14 betrieben werden. Die Auswerteschaltung 12 und die LED-Ansteuerung 14 sind mit einem Rechner 15 und auch untereinander verbunden.

Das vom Photoempfänger 4 empfangene Signal wird über einen Vorverstärker 16 einer Abtast- und Halteschaltung 17 zugeführt, in der die für unterschiedliche Wellenlängen sich ergebenden Intensitäten zwischengespeichert werden. In einer nachfolgenden Differenzschaltung 18 erfolgt eine Dunkelwert-Subtraktion, um den des Umgebungslichts zu eliminieren. Für die verschiedenen Wellenlängen erfolgt in einer weiteren Schaltung 19 ein Offsetabzug. Die derart aufbereiteten Signale gelangen dann zum Rechner 15, der daraus die normierte Perfusion ΔV/V ermittelt.

Die LED-Ansteuerung 14 enthält insbesondere den unterschiedlichen lichtemittierenden Dioden der Lichtquelle 13 zugeordnete Treiberstufen 20, die über eine vom Rechner 15 gesteuerte Vorstufenschaltung 21 angesteuert werden. Die Ansteuerung und die Auswertung erfolgt taktsynchron, wozu ein hier nicht näher dargestellter zentraler Taktgeber vorgesehen ist.

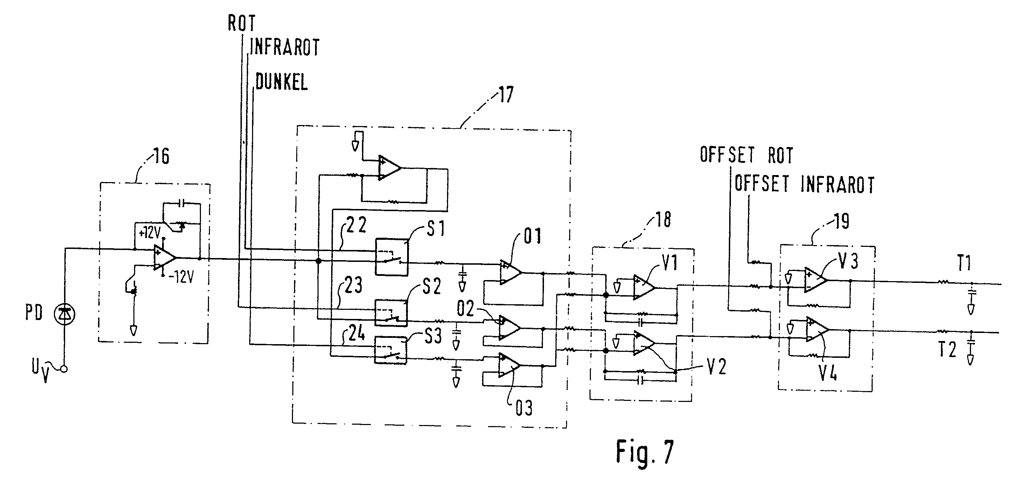

Der in Figur 7 dargestellte Ausschnitt zeigt die Auswerteschaltung 12 im Detail. Als Photoempfänger 4 dient eine Photodiode PD, die mit einer Vorspannung $U_V$ betrieben wird. Das Photodioden-Signal wird über den Vorverstärker 16 Schaltern S1 bis S3 zugeführt, die von der LED-Ansteuerungsschaltung an ihren Steuereingängen 22 bis 24 betätigt werden. Die Schalter S1 bis S3 sind mit positiven Eingängen dreier Operationsverstärker 01 bis 03 verbunden. Die Schaltung 18, die zur Subtraktion des Dunkelwertes dient, enthält zwei Differenzverstärker V1, V2 die ausgangsseitig mit weiteren Differenzverstärken V3, V4 der Schaltung 19 verbunden sind. Über Tiefpässe T1, T2 ist die Auswerteschaltung mit dem Rechner 15 verbunden.

Das erfindungsgemäße Verfahren ist nicht nur zur Bestimmung der Perfusion geeignet, sondern kann zum Beispiel auch als Stressindikator oder zur Indikation der Sensorpositionen herangezogen werden

**Ansprüche**

1. Verfahren zur Bestimmung der Perfusion durch Auswertung des von wenigstens einer Lichtquelle (13) ausgesandten und vom arteriellen Blut beeinflußten Lichts, unter Verwendung einer Meßeinrichtung, die die infolge einer arteriellen Blutvolumenänderung auftretende Intensitätsänderung (ΔI) des Lichts mißt, dadurch gekennzeichnet, daß die Messung der Lichtintensitätsänderung (ΔI) entweder
   - mit einer Wellenlänge am isobestischen Punkt oder
   - mit wenigstens zwei Wellenlängen ($\lambda_1$, $\lambda_2$), die sich voneinander und von der isobestischen Wellenlänge unterscheiden, vorgenommen wird, wobei aus der gemessenen Lichtintensitätsänderung (ΔI) unter Berücksichtigung der logarithmischen oder näherungsweise linearen Abhängigkeit zwischen der mittleren Dickenänderung (Δd) als Summe der partiellen Gefäßquerschnittsänderung eines optisch wirksamen, durchbluteten Gewebes (6) und der daraus resultierenden Lichtintensitätsänderung (ΔI) des vom Gewebe (6) reflektierten oder des das Gewebe (6) durchstrahlten Lichts die Dickenänderung ($\Delta d$) bzw. die Volumenänderung ($\Delta V/V$) - unter Annahme eines Wertes für die Hämoglobinkonzentration - ermittelt und als Perfusionsdicke ($\Delta$) bzw. als auf das Meßvolumen (V) des vom Blut durchströmten Gewebes (6) normierte Perfusion ($\Delta V/V$) angegeben wird.

2. Verfahren nach Anspruch 1, dadurch gekennzeichnet, daß die Lichtintensitätsänderung ($\Delta I$) von einem optoelektronischen Element (4) in eine Stromänderung umgewandelt und bezogen auf den maximalen Strom ($I_{max}$) als relative Stromänderung ($\Delta I/I_{max}$) ermittelt wird, und daß durch Einsetzen der bekannten Größen und Eliminieren der unbekannten Konzentration (c) aus der Gleichung

$$\Delta d = \frac{-\ln\left(1 - \frac{\Delta I}{I_{max}}\bigg|_{\lambda_i}\right)}{(E_{1i} \times c_1 + E_{2i} \times c_2 + \ldots + E_{ni} \times c_n)}$$

die Perfusionsdicke $\Delta d$ oder die normierte Perfusion $\Delta V/V$ ermittelt wird, wobei $\Delta I/I_{max}$ die relative Intensitätsänderung, bei der Wellenlänge $\lambda_i$, $c_1$ bis $c_n$ molare Konzentrationen für Hämoglobin, Oxyhämoglobin oder andere Stoffe sind, $E_{1i}$ bis $E_{ni}$ die Extinktionskoeffizienten für Hämoglobin, Oxyhämoglobin oder andere Stoffe bei der Wellenlänge $\lambda i$ sind.

3. Verfahren nach Anspruch 2, dadurch gekennzeichnet, daß bei einer Anzahl von unterschiedlichen Wellenlängen ($\lambda_i$) Messungen der relativen Intensitätsänderung ($\Delta I/I_{max}$) vorgenommen und daraus eine entsprechende Anzahl unbekannter Gleichungsparameter, wie Hämoglobinkonzentration, Sauerstoffsättigung und Perfusion ermittelt werden.

## Claims

1. Method for determining the perfusion by evaluation of light emitted by at least one light source (13) and influenced by the arterial blood, using a measuring device which measures the intensity variation ($\Delta I$) of the light caused by a change in the arterial blood volume,
   characterized in that measurement of the light intensity variation ($\Delta I$) is performed either
   - using one wavelength at the isobestic point or
   - using at least two wavelengths ($\lambda_1, \lambda_2$) which are different from each other and different from the isobestic wavelength, wherein the variation in thickness ($\Delta d$) or the volume variation ($\Delta V/V$) is determined - assuming a value of the haemoglobin concentration - from the measured light intensity variation ($\Delta I$) in view of the logarithmic or approximately linear relation between the medium variation in thickness ($\Delta d$) as the sum of the partial change of vessel sectional area of an optically active, circulated tissue (6) and the resulting variation in light intensity ($\Delta I$) of the light reflected by or transmitted through the tissue (6), and wherein such variation in thickness ($\Delta d$) or volume variation ($\Delta V/V$) is indicated as perfusion thickness ($\Delta d$) or as perfusion ($\Delta V/V$) normalized with respect to the measurement volume (V) of the tissue (6) supplied with by blood.

2. Method according to claim 1, characterized in that the light intensity variation ($\Delta I$) of an optoelectronic element (4) is converted into a current variation and determined as relative current variation ($\Delta I/I_{max}$) with respect to the maximum current ($I_{max}$) and that the perfusion thickness $\Delta d$ or the normalized perfusion $\Delta V/V$ is determined by means of substitution of known values and elimination of the unknown concentration (c) from the equation

$$\Delta d = \frac{-\ln\left(1 - \frac{\Delta I}{I_{max}}\bigg|_{\lambda_i}\right)}{(E_{1i} \times c_1 + E_{2i} \times c_2 + \ldots + E_{ni} \times c_n)}$$

wherein $\Delta I/I_{max}$ is the relative intensity variation at wavelength $\lambda_i$, $c_1$ to $c_n$ are molar concentrations of haemoglobin, oxyhaemoglobin or other substances, and $E_{1i}$ to $E_{ni}$ are the extinction coefficients of haemoglobin, oxyhaemoglobin or other substances at wavelength $\lambda_i$.

3. Method according to claim 2, characterized in that measurements of the relative intensity variation $(\Delta I/I_{max})$ are performed at a plurality of different wavelengths $(\lambda_i)$ and that these measurements are used to determine a corresponding number of unknown equation parameters, like haemoglobin concentration, oxygen saturation and perfusion.

## Revendications

1. Procédé de détermination de perfusion par évaluation de la lumière émise par au moins une source lumineuse (13) et influencée par du sang artériel, avec utilisation d'un dispositif de mesure qui mesure la variation d'intensité $(\Delta I)$ de la lumière se produisant par suite d'une variation de volume de sang artériel, caractérisé en ce que la mesure de la variation $(\Delta I)$ d'intensité de lumière est effectuée:
   - soit avec une longueur d'onde en un point isobestique,
   - soit avec au moins deux longueurs d'ondes $(\lambda_1, \lambda_2)$, qui se différencient l'une de l'autre et de la longueur d'onde isobestique, et, a partir de la variation d'intensité de lumière $(\Delta I)$ mesurée et en tenant compte de la relation logarithmique ou approximativement linéaire existant entre la variation moyenne d'épaisseur $(\Delta d)$ sous la forme d'une somme de la variation partielle de section de vaisseau d'un tissu (6) traversé par du sang et optiquement efficace et de la variation résultante d'intensité $(\Delta I)$ de la lumière réfléchie par le tissu (6) ou bien de la lumière traversant le tissu (6), la variation d'épaisseur $(\Delta d)$ ou bien la variation de volume $(\Delta V/V)$ est déterminée - en admettant une valeur pour la concentration d'hémoglobine - et elle est indiquée comme une épaisseur de perfusion $(\Delta)$ ou bien comme une perfusion $(\Delta V/V)$ normalisée en relation avec le volume de mesure (V) du tissu (6) traversé par le sang.

2. Procédé selon la revendication 1, caractérisé en ce que la variation d'intensité de lumière (I) est convertie par un élément optoélectronique (4) en une variation de courant et est définie, en relation avec le courant maximal $(I_{max})$, comme une variation relative de courant $(\Delta I/I_{max})$ et en ce que, en faisant intervenir les grandeurs connues et en éliminant la concentration inconnue (c), on obtient à partir de l'équation:

$$\Delta d = \frac{-\ln \left( 1 - \frac{\Delta I}{I_{max}} \Bigg|_{\lambda_i} \right)}{(E_{1i} \times c_1 + E_{2i} \times c_2 + \ldots + E_{ni} \times c_n)}$$

l'épaisseur de perfusion $\Delta d$ ou bien la perfusion normalisée $\Delta\ V/V$, $\Delta I/I_{max}$ désignant la variation relative d'intensité pour la longueur d'onde $\lambda_1$, $c_1$ à $c_n$ désignant des concentrations molaires pour l'hémoglobine, l'oxyhémoglobine ou d'autres substances, $E_i$ à $E_{ni}$ désignant les coefficients d'extinction concernant l'hémoglobine, l'oxyhémoglobine ou d'autres substances pour la longueur d'onde $\lambda_i$.

3. Procédé selon la revendication 2, caractérisé en ce qu'on effectue des mesures de la variation relative d'intensité $(\Delta I/I_{max})$ pour un certain nombre de longueurs d'ondes différentes $(\lambda_i)$ et on en déduit un nombre correspondant de paramètres inconnus de l'équation, comme la concentration en hémoglobine, la saturation en oxygène et la perfusion.

Fig.1

Fig.2

$I_{LED}$    $I_{REF}$

10   $I_{GEW}$

**Fig. 3**

I

$I_{max}$

$\Delta I$

t

**Fig. 4**

E

Hb

$HbO_2$

**Fig. 5**

λ

660 nm     950 nm

EP 0 293 504 B1

Fig. 6

10

Fig. 7

ROT
INFRAROT
DUNKEL

OFFSET ROT
OFFSET INFRAROT

PD

U_V

16    17    18    19

+12V
-12V

22    S1    01    V1    V3    T1
23    S2    02         V2    V4    T2
24    S3    03

EP 0 293 504 B1